# EUROPEAN PATENT APPLICATION

(11) **EP 2 955 224 A1**
(43) Date of publication of application: **16.12.2015**
(21) Application number: 14749363.9
(22) Date of filing: 03.02.2014
(51) Int. Cl.: C12N 9/12, A61K 38/17, B82Y 5/00, A61P 43/00

(54) **BIODEGRADABLE BIONANOPARTICLES FOR RELEASING THE GSE24-2 PEPTIDE, METHOD FOR THE PRODUCTION THEREOF, AND USE OF SAME**

(30) Priority: 05.02.2013 ES 201330131
(71) Applicant: Consejo Superior De Investigaciones Científicas (CSIC), 28006 Madrid (ES); Universidad Autónoma De Madrid (UAM), 28049 Madrid (ES); Universidad Del País Vasco/Euskal Herriko Unibertsitatea (UPV/EHU), 48940 Leioa (Vizcaya) (ES); Centro de Investigación Biomédica En Red de Enfermedades Raras, 46010 Valencia (ES)
(72) Inventor: PERONA ABELLON, Rosario, E-28029 Madrid (ES); PEDRAZ MUÑOZ, Jose Luis, E-48940 Leoia (Vizcaya) (ES); HERNANDEZ MARTIN, Rosa Maria, E-48940 Leoia (Vizcaya) (ES); IGARTUA OLAECHEA, Manuela, E-48940 Leoia (Vizcaya) (ES); EGUSQUIAGUIRRE MARTIN, Susana, E-48940 Leoia (Vizcaya) (ES); MANGUAN GARCIA, Cristina, E-46010 Valencia (ES)
(74) Representative: Pons Ariño, Angel
(86) International application number: PCT/ES2014/070072
(87) International publication number: WO 2014/122346

(57) **Abstract**

The invention relates to biodegradable PLGA bionanoparticles encapsulating the GSE24-2 peptide with telomerase activity, and to pharmaceutical, cosmetic and biotechnological compositions comprising same. The pharmaceutical compositions are useful for treating diseases presenting a telomerase deficiency, such as dyskeratosis congenita, and diseases presenting ageing characteristics, such as Werner syndrome, Rothmund Thompson syndrome, and other diseases where there is DNA damage such as ataxia telangiectasia. The relation also relates to the method for producing said bionanoparticles by means of a w/o/w double emulsion technique.

SEQUENCE LISTING

<110> Consejo Superior de Investigaciones Científicas
      CIBERER
      Universidad Autonoma de Madrid
      Universidad del País Vasco

<120> BIODEGRADABLE BIONANOPARTICLES FOR RELEASING THE GSE24-2 PEPTIDE, METHOD
FOR THE PRODUCTION THEREOF AND USE OF SAME

<130> GSE24.2

<160> 3

<170> BiSSAP 1.2

<210> 1
<211> 55
<212> PRT
<213> Homo sapiens


<400> 1
Gly Phe Ile Asn Leu Asp Lys Pro Ser Asn Pro Ser Ser His Glu Val
1               5                   10                  15
Val Ala Trp Ile Arg Arg Ile Leu Arg Val Glu Lys Thr Gly His Ser
            20                  25                  30
Gly Thr Leu Asp Pro Lys Val Thr Gly Cys Leu Ile Val Cys Ile Glu
        35                  40                  45
Arg Ala Thr Arg Leu Val Lys
    50                  55

<210> 2
<211> 18
<212> PRT
<213> Homo sapiens


<400> 2
Gly Phe Ile Asn Leu Asp Lys Pro Ser Asn Pro Ser Ser His Glu Val
1               5                   10                  15
Val Ala


<210> 3
<211> 25
<212> PRT
<213> Homo sapiens


<400> 3
His Ser Gly Thr Leu Asp Pro Lys Val Thr Gly Cys Leu Ile Val Cys
1               5                   10                  15
Ile Glu Arg Ala Thr Arg Leu Val Lys
            20                  25

## Description

### SECTOR OF THE ART

The present invention relates to the field of biomedical development and bionanotechnology, more specifically to the development of bionanoparticles for the preparation of medicines applicable in the pharmaceutical, cosmetic and biotechnological sector and for carrying drugs into a cell.

### STATE OF THE ART

The use of nanoparticles made of PLGA (poly lactic co-glycolic acid) has been disclosed relatively recently (since the end of 1970s). Nanoparticles are used either to carry the therapeutic substance to the target or as a protector of the encapsulated substance against potential degradation, as occurs in the case of peptides and proteins with therapeutic activity. Also, the nanoparticles allow for the sustained release of the encapsulated agent, making it possible to reduce the number of administrations, which is important in the case of chronic treatments. This helps to achieve greater compliance with the treatment on the part of the patient. In addition to the previously mentioned benefits, nanoparticles increase the solubility of not very soluble active ingredients, they protect the therapeutic agent against degradation, improve the bioavailability of the encapsulated agent, and make it possible to carry the agent to its place of action, thereby reducing the potential adverse toxic effects related to its systemic distribution. Additionally, nanoparticles can be administered via any route of administration, whether parenteral, oral, pulmonary, topical, etc.

Recently, a family of peptides derived from dyskerin, which are capable of reactivating telomerase activity, has been disclosed and even protected, along with the use of same as a medicine or pharmaceutical composition for the treatment of diseases with telomerase deficiency (Spanish priority patent application P200502511, entitled "Sequence of nucleotides and peptides GSE24.2 of dyskerin inducing telomerase activity, method for obtaining same, therapeutic compositions and their applications"). More specifically, peptide GSE24.2 is a peptide corresponding to the internal domain of the protein dyskerin which forms part of the telomerase complex. GE24.2 has the capacity to reactivate telomerase in pathological and physiological situations but not to enter the cell of its own accord. In diseases with telomerase deficiencies, the activity of the GSE24.2 peptide increases the viability of cells derived from patients with deficiencies in this activity although no descriptions are known of bionanoparticles which are capable of internalising and releasing these peptides in the cells of a mammal.

The encapsulation of peptides or proteins in biodegradable and biocompatible nanoparticle systems could improve their therapeutic potential while eliminating the limitations that condition use of same. Peptides or proteins can be adsorbed, bioconjugated, bound or encapsulated in nanoparticles. On being encapsulated in nanoparticles, their therapeutic activity can be increased, enabling their sustained release and carrying them to their place of action. At the same time, their stability is improved and bioavailability is increased.

However, during the encapsulation process, peptides and proteins can be subjected to determined conditions that can alter their therapeutic activity due to degradation, bundling, denaturalisation, or loss of their structure,; meaning that it is necessary to develop appropriate encapsulation techniques, optimised, specific and which do not affect their therapeutic and biological activity.

### DESCRIPTION OF THE INVENTION

### Brief description of the invention

One object of the invention consists of a bionanoparticles useful for the treatment of human diseases which present alterations of telomerase activity, hereinafter the bionanoparticles of the invention, wherein the bionanoparticle is made of poly lactic co-glycolic acid (PLGA) and comprises at least one GSE24.2 peptide whose amino acid sequence presents a percentage of homology of at least 95% with respect to SEQ ID NO1.

One particular object of the invention consists of a bionanoparticle of the invention wherein the GSE24.2 peptide is SEQ ID NO1.

Another particular object of the invention consists of a bionanoparticle of the invention which comprises polycations belonging, by way of illustration without limiting the scope of the invention, to the following group: polyethylenimine (PEI), chitosan (CS) and dextran (DX).

One particular embodiment of the invention consists of the bionanoparticle of the invention which comprises chitosan (CS) and the peptide GSE24.2 whose sequence is SEQ ID NO1.

Another object of the invention is the method for producing the bionanoparticle of the invention, hereinafter the method of the invention, which comprises the following stages:
a) emulsification through agitation of an aqueous solution of the GSE24.2 peptide which also comprises a protective agent against the peptide's denaturalisation, with a PLGA solution in organic solvent,
b) addition of the emulsion of a) to an aqueous solution containing a surfactant,
c) agitation of the above solution to obtain the W/O/W emulsion,
d) addition of the above mixture over an aqueous solution,
e) agitation until evaporation of the organic solvent and formation of the bionanoparticles,
f) recovery of the bionanoparticles resulting from the above solution,
g) washing of the bionanoparticles until eliminating the residues of the organic solvent and emulsifier, and
h) addition of a cryoprotectant.

Another particular object of the invention consists of the method of the invention wherein in stage a) the weight of the GSE 24.2 peptide with respect to the weight of the PLGA is between 0.25% and 20%.

Another particular embodiment of the invention consists of the method of the invention wherein the peptide GSE24.2 presents SEQ ID NO1.

Another particular object of the invention consists of the method of the invention wherein in stage a) the protective agent against the peptide's denaturalisation, which protects against the extreme conditions of the encapsulation process (contact with organic solvents, agitation, etc.) belongs, by way of illustration and without limiting the scope of the invention, to the following group: polyethylene glycol (PEG), bovine serum albumin (BSA) or human serum albumin (HSA).

Another particular object of the invention consists of the method of the invention wherein in stage a) the organic solvent belongs to, by way of illustration and without limiting the scope of the invention, the following group: dichloromethane, ethyl acetate, acetone, ethanol, acetonitrile, dimethyl sulfoxide, isopropyl formate or chloroform alone or combined.

One more particular embodiment of the invention consists of the method of the invention wherein in a more preferred embodiment polyvinyl alcohol (PVA) is used in a concentration of between 0.2% and 10% (p/v).

Another particular object of the invention consists of the method of the invention wherein in stage d) only water is added.

Another more particular embodiment of the invention consists of the method of the invention wherein in stage d) other organic solvents are added.

Another more particular embodiment of the invention consists of the method of the invention wherein in stage d) isopropanol or polyvinyl alcohol is added.

Another particular object of the invention consists of the method of the invention wherein in stage h) cryoprotectants are used fundamentally to avoid the bundling of the bionanoparticles on subjecting them to the subsequent process of lyophilisation.

Another more particular embodiment of the invention consists of the method of the invention wherein in stage h) trehalose is used as a cryoprotectant.

Another particular object of the invention consists of the method of the invention wherein a polycation is incorporated belonging, by way of illustration and without limiting the scope of the invention, to the following group: polyethylenimine (PEI), chitosan (CS) and dextran (DX).

Another object of the invention consists of the use of the bionanoparticle of the invention to prepare a pharmaceutical, cosmetic or biotechnological composition.

Another object of the invention consists of a pharmaceutical composition useful for the treatment of diseases such as dyskeratosis congenita, Werner syndrome, idiopathic pulmonary fibrosis, aplastic anaemia, hereinafter pharmaceutical composition of the invention, which comprises as an active ingredient at least the bionanoparticle of the invention, in a pharmaceutically effective quantity together with, optionally, one or more pharmaceutically acceptable carriers and/or adjuvants.

At the same time, another object of the invention consists of a pharmaceutical composition useful for the treatment of diseases that present ageing characteristics such as Werner syndrome, Rothmund Thompson syndrome, and other diseases where there is damage to DNA such as ataxia telangiectasia.

Another object of the invention consists of a cosmetic composition useful for the treatment of human skin, hereinafter the cosmetic composition of the invention, which comprises as the active ingredient at least the bionanoparticle of the invention, in an effective quantity, together with, optionally, one or more cosmetically acceptable carriers and/or adjuvants.

Another object of the invention consists of a biotechnological composition useful as a reagent to maintain mammal cells in culture, hereinafter the biotechnological composition of the invention, which comprises as a reagent at least the bionanoparticle of the invention, in an effective quantity, together with, optionally, one or more acceptable reagents and/or adjuvants.

Another object of the invention consists of the use of the bionanoparticle of the invention or of the pharmaceutical composition of the invention, hereinafter use of the pharmaceutical composition of the invention, in a method of treatment or prophylaxis of a mammal, preferably, a human being, affected by a disease associated with a deficiency in telomerase activity belonging, by way of illustration and without limiting the scope of the invention, to the following group: dyskeratosis congenita, Werner syndrome, idiopathic pulmonary fibrosis and aplastic anaemia, involving the administration of said bionanoparticle or therapeutic composition in an adequate dose that makes it possible to reduce or eliminate said disease. More preferably, said disease is dyskeratosis congenita.

Similarly, another object of the invention consists of the use of the bionanoparticle of the invention or of the pharmaceutical composition of the invention in a method of treatment or prophylaxis of a mammal, preferably, a human being, affected by a disease presenting ageing characteristics such as Werner syndrome, Rothmund Thompson syndrome, and other diseases where there is damage to DNA such as ataxia telangiectasia.

### Detailed description of the invention

The invention is based on the fact that the inventors have demonstrated that it is possible to obtain bionanoparticles of PLGA in which the GSE24.2 peptide has been encapsulated, capable of introducing said peptide not only into the cell of a mammal but also to reach the nucleus given that the particle is capable of releasing it inside the nucleus, therefore further allowing it to exert its biological activity. The peptide GSE24.2 is not capable of efficiently internalising in human cells of its own accord.

From the data obtained, it is possible to conclude that the peptide GSE24.2 has been successfully encapsulated in biodegradable nanoparticles. Also, the biological activity and structure of the GSE24.2 peptide are maintained after encapsulation and an acceptably high cell viability is also obtained. Finally, the nanoparticles of PLGA are efficiently internalised by the F9 cells and are capable of transporting it to the nucleus (Example 3).

On a separate note, it has been observed that the use of polycations, such as for example, polyethylenimine (PEI), chitosan (CS) and dextran (DX), has made it possible to obtain bionanoparticles with a greater efficiency in cellular uptake (between 30 and 60%) (Example 2). The positive charge of the surface of the bionanoparticles prepared with polycations favours entry of same into the cells by means of electrostatic interactions with the negatively charged cell membrane. The bionanoparticles can penetrate the cells through different mechanisms of endocytosis, the most common ones being clathrin-mediated and caveolae-mediated endocytosis. In the same way, they could be administered by the parenteral route.

These bionanoparticles can be applied as drugs or medicines in all those diseases which present deficient telomerase activity and for anti-ageing therapies in which the GSE24.2 peptide and other family-member peptides are considered appropriate. These diseases include dyskeratosis congenita, aplastic anaemia, idiopathic pulmonary fibrosis and for anti-ageing therapies in diseases such as Werner syndrome, Rothmund Thompson syndrome, and other syndromes where there is damage to DNA such as ataxia telangiectasia. It can also be applied as a therapy to maintain normal cells in culture and in treatments on epithelia such as the skin.

Thus, one object of the invention consists of a bionanoparticle useful for the treatment of human diseases presenting alterations of telomerase activity, hereinafter the bionanoparticle of the invention, wherein the bionanoparticle is made of poly lactic co-glycolic acid (PLGA) and comprises at least one GSE24.2 peptide whose amino acid sequence presents a percentage of homology of at least 95% with respect to SEQ ID NO1.

An expert in molecular biology, genetic engineering and biotechnology can develop new peptides such as the ones described in the present invention by means of adding other amino acids or artificial synthetic compounds. The first description of a GSE 24.2 peptide activating telomerase function was a fragment of 55 amino acids corresponding to a sequence which comprises two domains of dyskerin highly preserved in different species, called TRUB (Spanish priority patent application P200502511, entitled "Sequence of nucleotides and peptides GSE24.2 of dyskerin inducing telomerase activity, method for obtaining same, therapeutic compositions and their applications"), this peptide being the one that has been used as the model for embodying the bionanoparticle of the present invention. These peptides consisting of these TRUB domains increase telomerase activity just like the entire sequence of 55 amino acids initially described.

One particular object of the invention consists of a bionanoparticle of the invention wherein the peptide GSE24.2 is SEQ ID NO1.

Another particular object of the invention consist of a bionanoparticle of the invention wherein the peptide GSE24.2 is a fragment of SEQ ID NO1, preferably a fragment comprising at least one TRUB domain of dyskerin.

A more particular object of the invention consists of a bionanoparticle of the invention wherein the peptide GSE24.2 is a fragment of SEQ ID NO1 selected from the following group: SEQ ID NO2 and SEQ ID NO3 (include peptides of these domains disclosed in the first patent document).

Another particular object of the invention consists of a bionanoparticle of the invention which comprises polycations belonging, by way of illustration and without limiting the scope of the invention, to the following group: polyethylenimine (PEI), chitosan (CS) and dextran (DX).

One particular embodiment of the invention consists of the bionanoparticle of the invention comprising chitosan (CS) and the peptide GSE24.2 whose sequence is SEQ ID NO1.

One particular embodiment of the invention consists of the bionanoparticle of the invention comprising chitosan (CS) and the peptide GSE24.2 whose sequence is SEQ ID NO2 or SEQ ID NO3.

The term "human diseases presenting alterations of telomerase activity" as used in the present invention refers to a human disease belonging, by way of illustration and without limiting the scope of the invention, to the following group: dyskeratosis congenita, aplastic anaemia, idiopathic pulmonary fibrosis.

Similarly, the bionanoparticle of the invention can be used for the treatment of diseases presenting ageing characteristics such as Werner syndrome, de Rothmund Thompson syndrome, and other diseases where there is damage to DNA such as ataxia telangiectasia. The bionanoparticle of the invention can also be used as a biotechnological reagent for normal cells in culture and in treatments on epithelia, more specifically as a cosmetic treatment of the skin.

Another object of the invention is the method for producing the bionanoparticle of the invention, hereinafter the method of the invention, which comprises the following stages:
i) emulsification through agitation of an aqueous solution of the GSE24.2 peptide which also comprises a protective agent against the peptide's denaturalisation, with a PLGA solution in organic solvent,
j) addition of the emulsion of a) to an aqueous solution containing a surfactant,
k) agitation of the above solution to obtain the W/O/W emulsion,
l) addition of the above mixture over an aqueous solution,
m) agitation until evaporation of the organic solvent and formation of the bionanoparticles,
n) recovery of the bionanoparticles resulting from the above solution,
o) washing of the bionanoparticles until eliminating the remains of the organic solvent and emulsifier, and
p) addition of a cryoprotectant.

The formation of the bionanoparticles takes place following the extraction of the volatile solvent in which the polymer was solubilised towards the external aqueous phase.

Another particular object of the invention consists of the method of the invention wherein in stage a) the weight of the GSE 24.2 peptide with respect to the weight of the PLGA is between 0.25% and 20%.

Another particular object of the invention consists of the method of the invention wherein the GSE24.2 peptide is selected, by way of illustration and without limiting the scope of the invention, from the following group: SEQ ID NO1, SEQ ID NO2 and SEQ ID NO3.

Another particular embodiment of the invention consists of the method of the invention wherein the GSE24.2 peptide presents SEQ ID NO1.

Another particular object of the invention consists of the method of the invention wherein in stage a) the protective agent against the denaturalisation of the peptide which protects it against the extreme conditions of the encapsulation process (contact with organic solvents, agitation, etc.) belongs, by way of illustration and without limiting the scope of the invention, to the following group: polyethylene glycol (PEG), bovine serum albumin (BSA) or human serum albumin (HSA).

Another particular embodiment of the invention consists of the method of the invention wherein in stage a) the protective agent is PEG 400.

Another particular object of the invention consists of the method of the invention wherein in stage a) the organic solvent belongs by way of illustration and without limiting the scope of the invention, to the following group: dichloromethane, ethyl acetate, acetone, ethanol, acetonitrile, dimethyl sulfoxide, isopropyl formate or chloroform alone or combined.

Another particular embodiment of the invention consists of the method of the invention wherein in stage a) the weight of the PLGA with respect to the volume of dichloromethane is between 2.5% and 10%.

Another particular object of the invention consists of the method of the invention wherein in stage a) the agitation is carried out by sonication, although high pressure homogenisers or turbine agitators could be used.

Another particular embodiment of the invention consists of the method of the invention wherein in stage a) a first sonication is carried out of 15 to 60 seconds at a power of 50-100 W followed by a second sonication of 15-120 seconds at 50 - 100W.

Another particular object of the invention consists of the method of the invention wherein in stage b) the surfactant used is Tween 20, Tween 80, poloxamer, cetyl trimethyl Ammonium Bromide (CTAB), sodium cholate or polyvinyl alcohol (PVA).

It has been found that polyvinyl alcohol offers greater protection against coalescence, resulting in an emulsion with smaller droplets, giving rise to relatively uniform nanoparticle sizes. The concentration of PVA is a factor that affects the size of the nanoparticles, with a lower size for a greater percentage of PVA used.

Thus, one more particular embodiment of the invention consists of the method of the invention wherein in a more preferred embodiment polyvinyl alcohol (PVA) is used in a concentration of between 0.2% and 10% (p/v).

Another more particular embodiment of the invention consists of the method of the invention wherein the ratio of phases PLGA solution: PVA solution varies between 1:5 and 1: 40.

Another particular object of the invention consists of the method of the invention wherein in stage c) it is possible to agitate by means of a turbine agitator, high pressure homogeniser or sonication.

The choice of type of agitation influences the final size of the nanoparticle. Thus, the more intense the agitation, the lower the emulsion droplet size, which will in turn give rise to a lower particle size, once extraction of the solvent takes place and formation of the nanoparticles on precipitating the PLGA. In a more preferred embodiment of the method of the invention, in stage c) it is sonicated for a time of more than 20 seconds, preferably between 30 and 90 seconds, and more preferably during 60 seconds; and with a power of more than 50 W.

Another particular object of the invention consists of the method of the invention wherein in stage d) only water is added.

Another more particular embodiment of the invention consists of the method of the invention wherein in stage d) other organic solvents are added. The choice of solvent will influence the extraction of the organic solvent during the final stage of mechanical agitation.

Another more particular embodiment of the invention consists of the method of the invention wherein in stage d) isopropanol or polyvinyl alcohol is added.

Another more particular embodiment of the invention consists of the method of the invention wherein in stage e) the agitation may be magnetic or by means of a blade agitator. In this stage, a temperature increase to the 30°C-35°C range or application of a vacuum favours the solvent's evaporation.

Another particular object of the invention consists of the method of the invention wherein in stage f) the bionanoparticles of the invention are isolated by means of general filtration or centrifugation.

Another more particular embodiment of the invention consists of the method of the invention wherein centrifuging is carried out at less than 25,000 g.

Another particular object of the invention consists of the method of the invention wherein in stage g) the particles are washed in distilled water. It would also be possible to eliminate the remains of solvents and surfactants by means of dialysis and gel filtration but then more stages would be required to isolate the nanoparticles.

Another particular object of the invention consists of the method of the invention wherein in stage h) cryoprotectants are used fundamentally to avoid the bundling of the bionanoparticles on subjecting them to the subsequent process of lyophilisation. The most frequently used cryoprotectants are the sugars: trehalose, sucrose, glucose or mannitol although it would also be possible to use lactose, sorbitol, Aerosil (colloidal silicon dioxide), maltose, PVP (polyvinylpyrrolidone), fructose, dextran, glycerol, PVA (poly vinyl alcohol), glycine, HPβCD (hydroxypropyl-β-cyclodextrin) or gelatine.

Another more particular embodiment of the invention consists of the method of the invention wherein in stage h) trehalose is used as the cryoprotectant. Trehalose is one of the most widely used cryoprotectants due to its numerous benefits: low hygroscopicity or the absence of internal hydrogen bonds, which allows for the formation of more flexible hydrogen links with the nanoparticles during lyophilisation. Furthermore, trehalose has a very low chemical reactivity and a high vitreous transition temperature. It has been verified that the use of trehalose in a percentage of between 2.5 - 10% is capable of protecting the bionanoparticles of the invention during their subsequent freezing and lyophilisation. Furthermore, it also increases the stability of the nanoparticles during storage of same.

Another particular object of the invention consists of the method of the invention wherein the bionanoparticles are frozen and lyophilised to maintain their stability and avoid release of the peptide before they are administered.

Another particular object of the invention consists of the method of the invention wherein a polycations is incorporated belonging, by way of illustration and without limiting the scope of the invention, to the following group: polyethylenimine (PEI), chitosan (CS) and dextran (DX).

In a preferred embodiment of the method of the invention the polycation CS and the polycation DX are dissolved in the aqueous solution of PVA in stage d). In a preferred embodiment of the method of the invention the polycation PEI is dissolved in dichloromethane together with the PLGA in stage a).

Another object of the invention consists of the use of the bionanoparticle of the invention in the preparation of a pharmaceutical, cosmetic or biotechnological composition.

Another object of the invention consists of a pharmaceutical composition useful for the treatment of diseases such as dyskeratosis congenita, Werner syndrome, idiopathic pulmonary fibrosis, aplastic anaemia, hereinafter the pharmaceutical composition of the invention, which comprises as the active ingredient at least the bionanoparticle of the invention, in a pharmaceutically effective quantity together with, optionally, one or more pharmaceutically acceptable carriers and/or adjuvants.

Similarly, another object of the invention consists of the pharmaceutical composition useful for the treatment of diseases presenting ageing characteristics such as Werner syndrome, Rothmund Thompson syndrome, and other diseases where there is damage to DNA such as ataxia telangiectasia.

Another object of the invention consists of a cosmetic composition useful for the treatment of human skin, hereinafter the cosmetic composition of the invention, which comprises as the active ingredient at least the bionanoparticle of the invention, in an effective quantity, together with, optionally, one or more cosmetically acceptable carriers and/or adjuvants.

Another object of the invention consists of a biotechnological composition useful as a reagent for maintaining mammal cells in culture, hereinafter the biotechnological composition of the invention, which comprises as the reagent at least the bionanoparticle of the invention, in an effective quantity together with, optionally, one or more acceptable reagents and/or adjuvants.

Another object of the invention consists of the use of the bionanoparticle of the invention or of the pharmaceutical composition of the invention, hereinafter the use of the pharmaceutical composition of the invention, in a method of treatment or prophylaxis of a mammal, preferably a human being affected by a disease associated with a deficiency in telomerase activity belonging, by way of illustration and without limiting the scope of the invention, to the following group: dyskeratosis congenita, Werner syndrome, idiopathic pulmonary fibrosis and aplastic anaemia, involving the administration of said bionanoparticle or therapeutic composition in an adequate dose which makes it possible to reduce or eliminate said disease. More preferably, said disease is dyskeratosis congenita.

Similarly, another object of the invention consists of the use of the bionanoparticle of the invention or of the pharmaceutical composition of the invention in a method of treatment or prophylaxis of a mammal, preferably a human being affected by a disease presenting ageing characteristics such as Werner syndrome, Rothmund Thompson syndrome, and other diseases where there is damage to DNA such as ataxia telangiectasia.

In another preferred embodiment of the present invention, the method of treatment defined above is characterised in that the bionanoparticle or the pharmaceutical composition comprises a second active ingredient.

### DESCRIPTION OF THE DRAWINGS

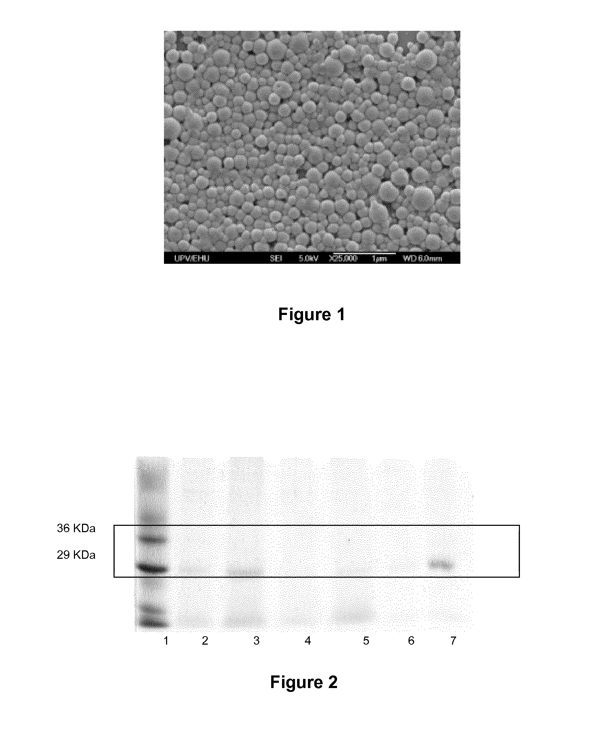

**Figure 1****.- Morphology of the bionanoparticles of PLGA loaded with the peptide GSE24.2 obtained by means of scanning electron microscopy (SEM).** The microphotograph shows small spherical particles, with a smooth and uniform surface, with a particle size in the nanometric range.
**Figure 2****.- Structural integrity studies by means of SDS-PAGE of the bionanoparticles of the invention.** The structural integrity studies by means of SDS-PAGE of the bionanoparticles of PLGA loaded with the peptide GSE24.2 do not show damage to the peptide structure during the encapsulation process. Lane 1: standard molecular weight; lanes 2-7: peptide GSE 24.2 extracted from the bionanoparticles (approximately 10 □g). The peptide samples extracted from the bionanoparticles present a characteristic band near to the 29 kDa band of the standard molecular weight, corresponding to the molecular weight of the peptide GSE24.2.

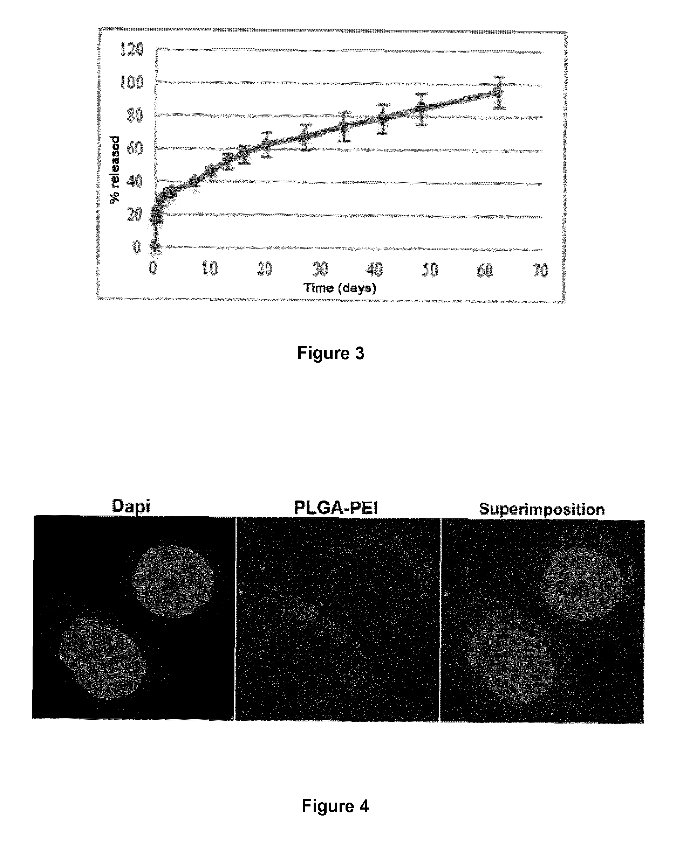

**Figure 3****.- Release profile in vitro of nanoparticles of PLGA loaded with the peptide GSE24.2.** The bionanoparticles loaded with the peptide GSE24.2 have been incubated in a culture medium and at different times the quantity of released peptide has been measured. The figure shows the released quantity represented as a percentage of the maximum released following 60 hours of incubation. The bionanoparticles present a two-phase release profile, with an initial release of 25% of the peptide GSE 24.2, also referred to as the 'burst effect, corresponding to the peptide adsorbed to the surface of the nanoparticles, followed by a second stage of sustained release, until achieving an almost total release of the peptide (close to 100%) during the 60 day duration of the test. The results shown have been similar using the different compositions of bionanoparticles except those loaded with heparin, which on account of its toxicity have been discarded for all other studies.
**Figure 4****.- Intracellular localisation of bionanoparticles of PLGA loaded with the peptide GSE24.2.** The HeLa cells were treated with bionanoparticles of PLGA-PEI and PLGA (not shown) marked with fluorescein isothiocyanate at a concentration of bionanoparticles of 1 mg/ml and following 24 hours fluorescence was measured in a confocal microscope. A photograph is shown of the PLGA-PEI particles. DAPI (4',6-diamidino-2-phenylindole) is the nuclear dye with the fluorophore that binds to the DNA (emission wavelength 455 nm), PLGA-PEI represents the fluorescence (fluorescein emission at 515 nm) of the bionanoparticles and the superimposition of the two images.

### EXAMPLES OF EMBODIMENT OF THE INVENTION

### Example 1.- Preparation of bionanoparticles of the invention of PLGA (poly lactic co glycolic acid) and GSE24.2 by means of the solvent evaporation technique using a double emulsion system (w/o/w).

This example describes the preparation of bionanoparticles of the peptide GSE24.2 (SEQ ID NO1) with PLGA (poly lactic co-glycolic acid) with the solvent evaporation technique using a double emulsion system (w/o/w) in conditions which maintain the functional properties of said peptide. More specifically, the bionanoparticles were prepared emulsifying an aqueous solution of the peptide GSE24.2 at 0.5% (p/v) with a solution of PLGA (Resomer^{®} RG503) at 5% (p/v) in 2 ml of dichloromethane, containing PEG 400 at 2.5% (v/v), by means of the application of ultrasounds during 30 seconds at a power of 50W (Branson Sonifier^{®}).

This primary emulsion was added to 10 ml of an aqueous solution 5% (p/v) of polyvinyl alcohol (PVA) and it was sonicated again during 60 seconds at a power of 50W to obtain the w/o/w emulsion. Finally, this second emulsion was poured over an aqueous solution at 2% (v/v) of isopropanol and the mix was subjected to mechanical agitation during 2 hours. The formation of the bionanoparticles of the invention GSE24.2-PLGA took place following the extraction of the volatile solvent in which the polymer was solubilised towards the external alcohol phase.

Finally, the bionanoparticles GSE24.2-PLGA suspended in the aqueous phase were isolated by means of centrifuging at 25,000 g and washed three times in distilled water to eliminate residues of the organic solvent and emulsifier. Immediately after the last centrifuging and after eliminating the supernatant 1 ml of an aqueous solution of trehalose at 5% (p/v) was added. Next, they were frozen during 24 hours at -20°C and 24 hours at -80°C, and lyophilised for correct storage and subsequent use.

### Example 2.- Method for producing the cationic bionanoparticles

The above preparation method was followed to prepare the cationic bionanoparticles GSE24.2-PLGA, incorporating different polycations such as polyethylenimine (PEI), chitosan (CS) and dextran (DX). The CS and DX were dissolved in an aqueous solution of PVA whereas PEI was dissolved in dichloromethane, together with the PLGA.

As can be appreciated from Table 1, the size of the bionanoparticles of GSE24.2-PLGA is in the region of 220 nm, with the size of 50% of the bionanoparticles having a narrow size distribution (polydispersity index PDI < 1). With regards to the surface charge of the bionanoparticles, it was observed that the addition of polycations gives rise to a positive charge except in the case of CS, which acquires a positive charge in an acid medium.

**Table I.- Characterisation of the different formulations of prepared nanoparticles (PDI: polydispersity index, Potential ζ: surface charge, EE: encapsulation efficiency, SAP: surface adsorbed protein).**

| **Bionanoparticle formulation** | **Size (nm)** | **PDI** | **Potential** ζ | **EE (%)** | **SAP (%)** |
|---|---|---|---|---|---|
| **PLGA** | 231,2 | 0,126 | -20,1 | 60,27 | 28,64 |
| **PLGA-CS** | 243,9 | 0,151 | -3,05 | 18,89 | 9,36 |
| **PLGA-PEI** | 221,9 | 0,144 | 30,4 | 86,42 | 23,33 |
| **PLGA-DX** | 226,2 | 0,095 | 24,4 | 30,48 | 4,32 |

The nanoparticles were characterised in terms of size and potential Z, by dynamic light scattering and Laser Doppler Micro-Electrophoresis, respectively. The morphology of the nanoparticles was analysed by means of scanning electron microscopy (SEM).

### Example 3.- Biological activity of the nanoparticles of the invention.

The efficiency of the encapsulation of the peptide GSE24.2 encapsulated in the bionanoparticles of the invention, the percentage of the peptide GSE24.2 adsorbed to the surface and the release in vitro were evaluated using a micro-BCA kit which assesses the quantity of protein.

As can be observed from the SEM images (Figure 1) small spherical particles are obtained in the nanometric range which are similar to those obtained in the absence of peptide.

According to the structural integrity studies using SDS-PAGE (Figure 2), no changes in the integrity of the peptide are observed as there are no changes in the peptide's migration in the electrophoresis gels after the encapsulation process, in other words, it is not degraded during this process. Therefore, the biological activity of the GSE24.2 incorporated into the nanoparticles ought to be maintained.

The cytotoxicity of the bionanoparticles of PLGA loaded with the peptide GSE24.2 was tested in HeLa and MEF cells. The nanoparticles were deposited in culture plates of MEF cells (mouse embryonic fibroblasts) or HeLa at growing concentrations (0; 1.25; 1.875, 2.5 mg/ml and 5 mg/ml). After 72 hours, cell viability was determined using the crystal violet method. Table II shows the inhibitory dose 50%, in other words, the dose of bionanoparticles of the invention in which an inhibition of viability is observed of 50%. It is shown for different prepared formulations, including very toxic ones such as PLGA-heparin and which were discarded for subsequent biological testing. The tested bionanoparticles were: PLGA, PLGA-CS, PLGA-PEI. PLGA-DX and PLGA-heparin. The bionanoparticles prepared with PLGA only barely show any toxicity, as in the case of the bionanoparticles prepared with PLGA-CS. However, the bionanoparticles of PLGA-PEI are somewhat more toxic as viability diminishes considerably on increasing the concentration of bionanoparticles administered. The most toxic ones are those prepared with PLGA-DX which also show a fairly toxic profile, and the ones of PLGA-heparin have also proven to be very toxic.

**Table II- Toxicity study in vitro of the bionanoparticles of the invention.**

| **Formulation** | **Viability IC50%- MEF** | **Viability IC50%-HeLa** |
|---|---|---|
| **PLGA** | 2.5 | 5 |
| **PLGA-CS** | >5 | >5 |
| **PLGA-PEI** | 1.4 | 1.7 |
| **PLGA-DX** | 0.5 | 0.35 |
| **PLGA-Heparin** | <0.01 | <0.01 |

As demonstrated in the release studies *in vitro* (Figure 3) the bionanoparticles present a two-phase profile with an initial release of 25% of the total encapsulated peptide, corresponding to the so-called 'burst' effect. This first phase is followed by a second phase of sustained release achieving values near to 100% of the total of GSE24.2 loaded in the bionanoparticles. Therefore, these bionanoparticles represent a continued release system at least during 60 hours. Similar results were obtained for all the bionanoparticles tested, except for those of PLGA-heparin which were discarded due to their high toxicity.

For these determinations of biological activity, the least toxic bionanoparticles were used, which best internalised, in other words PLGA and PLGA-PEI. The bioactivity in vitro of the peptide GSE24.2 encapsulated in the bionanoparticles of PLGA was determined by measuring the capacity of the GSE24.2 to rescue the DNA damage present in F9A353V cells. These cells express the most frequent DKC1 gene mutation in patients with dyskeratosis congenita. The DNA damage was measured by evaluating by means of western blot the signal obtained with an antibody against the histone H2AX (Defects in mTR stability and telomerase activity produced by the Dkc1 A353V mutation in dyskeratosis congenita are rescued by a peptide from the dyskerin TruB domain. Clin Transl Oncol. 2012 Oct; 14 (10): 755-63). Previous experiments demonstrate that the transfection of the peptide GSE24.2 using a lipid compound reduces the signal of the histone H2AX. The results have been obtained by incubating with A353V cells the equivalent of 15 µg of the peptide GSE24.2 released from the bionanoparticles. As can be appreciated from Table 3, the peptide GSE24.2 encapsulated in the bionanoparticles of PLGA preserves its biological activity *in vitro* rescuing the DNA damage induced by the deficient telomerase activity of the A353V cells.

**Table 3.- Bioactivity of the bionanoparticles of the invention**

| **Formulation of the bionanoparticle of GSE24.2** | **% rescued DNA damage** |
|---|---|
| **PLGA** | 20.09 ± 0.07 |
| **PLGA-PEI** | 38.21 ± 0.11 |

Further, the addition of PEI (PLGA-PEI) slightly increases the DNA damage rescued of the peptide GSE24.2, probably because its positive charge on the surface of the bionanoparticle can increase its internalisation (Table 3).

To verify whether the particles of PLGA loaded with GSE24.2 were internalised efficiently in the cells, the intracellular localisation was studied in F9 cells, (Figure 4). During the first 24 hours of incubation the results of the confocal microscopy indicated that the bionanoparticles were in the cells, reaching the nucleus (Figure 4). The smaller the bionanoparticles, the better they are internalised in the cells, and the better they reach the nuclei. This range of sizes is fairly adequate for the purpose of the experiments, as they are sizes which allow the cells to be penetrated and to reach the nuclei. The acceptable range for the bionanoparticles would be between 100 -500 nm, which is the range in which the bionanoparticles of PLGA obtained in example 1 are, approximately about 220 nm.

## Claims

1. Bionanoparticle useful for the treatment of human diseases presenting alterations of telomerase activity **characterised in that** the bionanoparticle is made of poly lactic co-glycolic acid (PLGA) and **in that** it comprises at least one peptide GSE24.2 whose amino acid sequence presents a percentage of homology of at least 95% with respect to SEQ ID NO1.

2. Bionanoparticle according to claim 1 **characterised in that** the peptide GESE24.2 is SEQ ID NO1.

3. Bionanoparticle according to claims 1 and 2 **characterised in that** the peptide GESE24.2 is a fragment of SEQ ID NO1, preferably a fragment comprising at least one TRUB domain of dyskerin.

4. Bionanoparticle according to claim 3 **characterised in that** the peptide GESE24.2 belongs to the following group: SEQ ID NO2 and SEQ ID NO3.

5. Bionanoparticle according to any of claims 1 to 4 **characterised in that** it comprises polycations.

6. Bionanoparticle according to claim 5 **characterised in that** the polycation belongs to the following group: polyethylenimine (PEI), chitosan (CS) and dextran (DX).

7. Bionanoparticle according to claim 1 **characterised in that** it comprises chitosan (CS) and the peptide GSE24.2 whose sequence is SEQ ID NO1.

8. Bionanoparticle according to claim 1 **characterised in that** it comprises chitosan (CS) and the peptide GSE24.2 whose sequence is SEQ ID NO2 or SEQ ID NO3.

9. Method for producing the bionanoparticle according to any of claims 1 to 8 **characterised in that** it comprises the following stages:
a) emulsification through agitation of an aqueous solution of the GSE24.2 peptide which also comprises a protective agent against the peptide's denaturalisation, with a PLGA solution in organic solvent,
b) addition of the emulsion of a) to an aqueous solution containing a surfactant,
c) agitation of the above solution to obtain the W/O/W emulsion,
d) addition of the above mixture over an aqueous solution,
e) agitation until evaporation of the organic solvent and formation of the bionanoparticles,
f) recovery of the bionanoparticles resulting from the above solution,
g) washing of the bionanoparticles until eliminating the remains of organic solvent and emulsifier, and
h) addition of a cryoprotectant.

10. Method according to claim 9 **characterised in that** in the stage a) the weight of the peptide GSE 24.2 with respect to the weight of the PLGA is between 0.25% and 20%.

11. Method according to either of claims 9 or 10 **characterised in that** the peptide GSE 24.2 is selected from the following group: SEQ ID NO1, SEQ ID NO2 and SEQ ID NO3.

12. Method according to claims 9 to 11 **characterised in that** the peptide GSE 24.2 presents SEQ ID NO1.

13. Method according to any of claims 9 to 12 **characterised in that** in stage a) the protective agent belongs to the following group: polyethylene glycol (PEG), bovine serum albumin (BSA) or human serum albumin (HSA).

14. Method according to any of claims 9 to 13 **characterised in that** in stage a) the protective agent is PEG 400.

15. Method according to any of claims 9 to 14 **characterised in that** in stage a) the organic solvent belongs to the following group: dichloromethane, ethyl acetate, acetone, ethanol, acetonitrile, dimethyl sulfoxide, isopropyl formate or chloroform alone or combined.

16. Method according to any of claims 9 to 15 **characterised in that** in stage a) the weight of PLGA with respect to the volume of dichloromethane is between 2.5% and 10%.

17. Method according to any of claims 9 to 16 **characterised in that** in stage a) agitation is carried out through sonication, turbine agitators, or high pressure homogenisers.

18. Method according to any of claims 9 to 17 **characterised in that** in stage a) a first sonication is carried out of 15 to 60 seconds at a power of 50-100 W followed by a second sonication of 15-120 seconds at 50 - 100W.

19. Method according to any of claims 9 to 18 **characterised in that** in stage b) the surfactant used is Tween 20, Tween 80, poloxamer, cetyl-trimethyl Ammonium Bromide (CTAB), sodium cholate or polyvinyl alcohol (PVA).

20. Method according to any of claims 9 to 19 **characterised in that** in stage b) polyvinyl alcohol (PVA) is used in a concentration of between 0.2% and 10% (p/v).

21. Method according to any of claims 9 to 20 **characterised in that** in stage b) the ration of the phases PLGA solution: PVA solution varies between 1:5 and 1: 40.

22. Method according to any of claims 9 to 21 **characterised in that** in stage c) agitation can be carried out by means of a turbine agitator, high pressure homogenisers or sonication.

23. Method according to any of claims 9 to 22 **characterised in that** in stage c) sonication is carried out during a time of more than 20 seconds, preferably between 30 and 90 seconds, and more preferably during 60 seconds; and with a power of more than 50 W.

24. Method according to any of claims 9 to 23 **characterised in that** in stage d) only water is added.

25. Method according to any of claims 9 to 24 **characterised in that** in stage d) other organic solvents are added.

26. Method according to claim 25 **characterised in that** in stage d) isopropanol or polyvinyl alcohol are added.

27. Method according to any of claims 9 to 26 **characterised in that** in stage e) agitation can be magnetic or by means of a blade agitator.

28. Method according to any of claims 9 to 27 **characterised in that** in stage f) the bionanoparticles of the invention are isolated by means of general filtration or centrifugation.

29. Method according to claim 28 **characterised in that** centrifuging takes place at less than 25,000 g.

30. Method according to any of claims 9 to 29 **characterised in that** in stage g) the particles are washed in distilled water.

31. Method according to any of claims 9 to 30 **characterised in that** in stage h) cryoprotectants are used belonging to the following group of sugars: trehalose, sucrose, glucose, mannitol, lactose, sorbitol, Aerosil, maltose, PVP (poly(vinyl pyrrolidone)), fructose, dextran, glycerol, PVA (polyvinyl alcohol), glycine, HPβCD (hydroxypropyl-β-cyclodextrin) or gelatine.

32. Method according to claim 31 **characterised in that** in stage h) trehalose is used as the cryoprotectant, in a percentage of between 2.5 - 10%.

33. Method according to any of claims 9 to 32 **characterised in that** a polycation is incorporated.

34. Method according to claim 33 **characterised in that** the polycation belongs to the following group: polyethylenimine (PEI), chitosan (CS) and dextran (DX).

35. Method according to claim 34 **characterised in that** the polycation CS or the polycation DX are dissolved in the aqueous solution of PVA in stage d).

36. Method according to claim 34 **characterised in that** the polycation PEI is dissolved in dichloromethane together with the PLGA in stage a).

37. Use of the bionanoparticle according to any of claims 1 to 8 for the preparation of a pharmaceutical, cosmetic or biotechnological composition.

38. Pharmaceutical composition useful for the treatment of diseases such as dyskeratosis congenita, Werner syndrome, idiopathic pulmonary fibrosis, aplastic anaemia, hereinafter the pharmaceutical composition of the invention **characterised in that** it comprises as the active ingredient at least the bionanoparticle according to any of claims 1 to 8, in a pharmaceutically effective quantity together with, optionally, one or more pharmaceutically acceptable carriers and/or adjuvants.

39. Pharmaceutical composition useful for the treatment of diseases presenting ageing characteristics such as Werner syndrome, Rothmund Thompson syndrome, and other diseases where there is damage to DNA such as ataxia telangiectasia, **characterised in that** it comprises as the active ingredient at least the bionanoparticle according to any of claims 1 to 8, in a pharmaceutically effective quantity, together with, optionally, one or more pharmaceutically acceptable carriers and/or adjuvants.

40. Cosmetic composition useful for the treatment of human skin **characterised in that** it comprises as the active ingredient at least the bionanoparticle of the invention according to any of claims 1 to 8 in an effective quantity, together with, optionally, one or more cosmetically acceptable carriers and/or adjuvants.

41. Biotechnological composition useful as a reagent for maintaining mammal cells in culture **characterised in that** it comprises as the reagent at least the bionanoparticle according to any of claims 1 to 8 in an effective quantity, together with, optionally, one or more acceptable reagents and/or adjuvants.

42. Use of the bionanoparticle according to any one of claims 1 to 8 or of the pharmaceutical composition according to any one of claims 38 and 39 in a method of treatment or prophylaxis of a mammal, preferably a human being, affected by a disease associated with deficient telomerase activity belonging to the following group: dyskeratosis congenita, Werner syndrome, idiopathic pulmonary fibrosis and aplastic anaemia, involving the administration of said bionanoparticle or pharmaceutical composition in an adequate dose which makes it possible to reduce or eliminate said disease.

43. Use of the bionanoparticle according to claim 42 **characterised in that** the disease is dyskeratosis congenita.

44. Use of the bionanoparticle according to any of claims 1 to 8 or of the pharmaceutical composition according to any one of claims 38 and 39 in a method of treatment or prophylaxis of a mammal, preferably a human being, affected by a disease presenting ageing characteristics such as Werner syndrome, Rothmund Thompson syndrome, and other diseases where there is damage to DNA such as ataxia telangiectasia.

45. Use according to any one of claims 42 to 45 **characterised in that** the bionanoparticle or the pharmaceutical composition comprises a second active ingredient.
